# EUROPEAN PATENT SPECIFICATION

(11) **EP 0 955 969 B1**
(45) Date of publication and mention of the grant of the patent: **08.10.2003**
(21) Application number: 96923129.9
(22) Date of filing: 28.06.1996
(51) Int. Cl.: A61F 7/12, A61B 18/08

(54) **DEVICE FOR LOCAL HEAT TREATMENT OF TISSUE**
VORRICHTUNG ZUR ÖRTLICHEN WÄRMEBEHANDLUNG VON GEWEBE
DISPOSITIF DE TRAITEMENT THERMIQUE LOCAL DES TISSUS

(30) Priority: 07.07.1995 SE 9502523
(43) Date of publication of application: 17.11.1999
(73) Proprietor: Prostalund Operations AB, 226 60 Lund (SE)
(72) Inventor: BOLMSJÖ, Magnus, S-223 40 Lund (SE)
(74) Representative: Asketorp, Göran
(86) International application number: SE9600800
(87) International publication number: WO97002794

(56) References cited:
- WO-A-96/15740
- US-A- 4 949 718

## Description

The invention concerns a device for heat treatment of body tissue according to patent claim 1.

Certain conditions of illness with unnatural growth of body tissue are successfully managed by the use of heat treatment. The tissue is heated to such an extent that the tissue dies. Certain types of cancer and benign prostate hyperplasia (BPH) are examples of such conditions of illness. During treatment certain parts of the tissue are to be treated whereas others must or should be protected. Conditions of illness primarily considered are those extant in tissues around cavities in the body. In addition to examples given above, the following conditions of illness should be mentioned: cancer in esophagus, trachea, ureter and intestines.

Similar conditions of illness may also appear in animals, for which equivalent treatment can be applied. Above all, it is the treatment of domestic animals and pets, such as dogs, that might be considered.

### STATE OF THE ART

Various devices may be used for the purpose of producing heat. Both laser heating devices and microwave and RF heating devices are commonly used. From US-A-5,257,977 a technology is known, according to which a treatment catheter is equipped with a fluid receptacle. The receptacle is flexible and communicates, via channels through the catheter, with a heating device provided external to the body and catheter. A fluid is heated in the heating device and is circulated through the channels and the receptacle, which is expanded to some extent for better contact against the tissue. Temperature increase in the receptacle also results in heating of the surrounding tissue. The treatment is influenced by temperature control of the circulating fluid.

Since the channels pass through such tissue that is not to be treated, they have to be heat insulated. According to US-A-5,257,977 heat insulation is provided through gas-filled spaces surrounding the channels. The function of heat insulation is very essential. Great care and significant costs therefore have to be spent on this part of the treatment catheter. Another disadvantage with respect to the device of US-A-5,257,977 is that it is difficult to attain desirable temperature control because of the relatively large distance between the heating device and the area of treatment.

WO-A-9615740 discloses a device for heat treatment of prostate gland tissue comprising a catheter provided with a central tube and an elastic balloon surrounding said tube. One end of said tube is provided with a valve enabling discharge of gas from the interior of the balloon. A heating element can be provided inside the balloon.

### SUMMARY OF THE INVENTION

According to the invention a treatment catheter is provided having an expandable receptacle in accord with the preamble of claim 1. A heating device is provided adjacent to the receptacle for heating the fluid contained in the receptacle. The fluid remains in the receptacle during the entire treatment. Thus, there is no need of heat insulated channels extending through the catheter.

According to one aspect of the invention, the temperature is measured both in the fluid and in the catheter outside of the area of treatment so as to ensure achievement of the desired treatment temperatures.

According to another aspect a mechanical means is provided in the receptacle for expansion of the receptacle in conjunction with the treatment.

In yet another aspect a so called stent is provided over the receptacle during insertion of the treatment catheter into the body. The stent is then left in place to maintain passage through the area of treatment.

### BRIEF DESCRIPTION OF THE DRAWINGS

The invention will now be described in more detail with the aid of exemplary embodiments and with reference to the accompanying drawings, in which:
- FIG 1: is a side view, partially in section, of one treatment catheter according to the preamble of claim 1,
- FIG 2: is an enlarged side view, partially in section, of an embodiment according to the invention,
- FIG 3: is a side view, partially in section, of a treatment catheter illustrating some special features;
- FIG 4: is an enlarged side view, partially in section, of a treatment catheter illustrating other special features, and
- FIG 5: is a cross-sectional view taken along line V - V in Fig. 4.

### DESCRIPTION

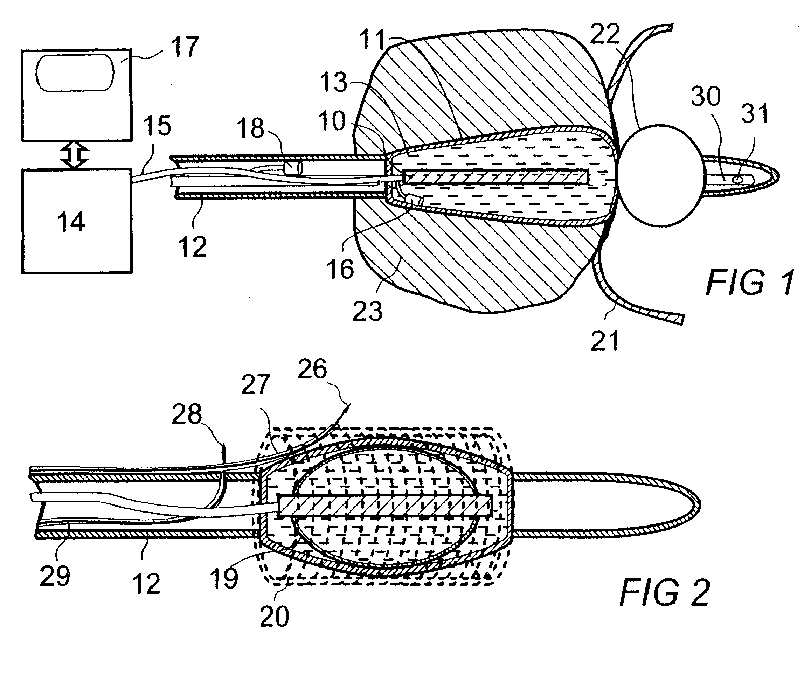

In Figure 1 there is shown a device, according to the preamble of claim 1, comprising a treatment catheter 12. In a portion near its first end the catheter is equipped with a receptacle 11 having a flexible and expandable wall. In a first embodiment the receptacle 11 is completely closed and contains a certain volume of a fluid 13 having appropriate heat transfer characteristics. Silicone oil and water are examples of such a fluid. The balloon 11 is made of elastic silicone or other material with corresponding elastic properties, for instance latex.

The treatment catheter 12 in Fig. 1 is inserted into and through the urethra so that a tip of the treatment catheter 12 enters the urine bladder 21. To make the treatment catheter 12 stay in place during treatment a balloon 22 adjacent to the tip of the treatment catheter 12 is expanded in a conventional way through a channel provided in the treatment catheter 12. In the shown treatment mode the receptacle 11 is positioned in the prostate gland 23. The purpose of the treatment is to heat the tissue in the prostate gland, in the environment surrounding the urethra, to cause tissue death in a defined partial volume of the prostate gland from the urine bladder neck down towards the apex.

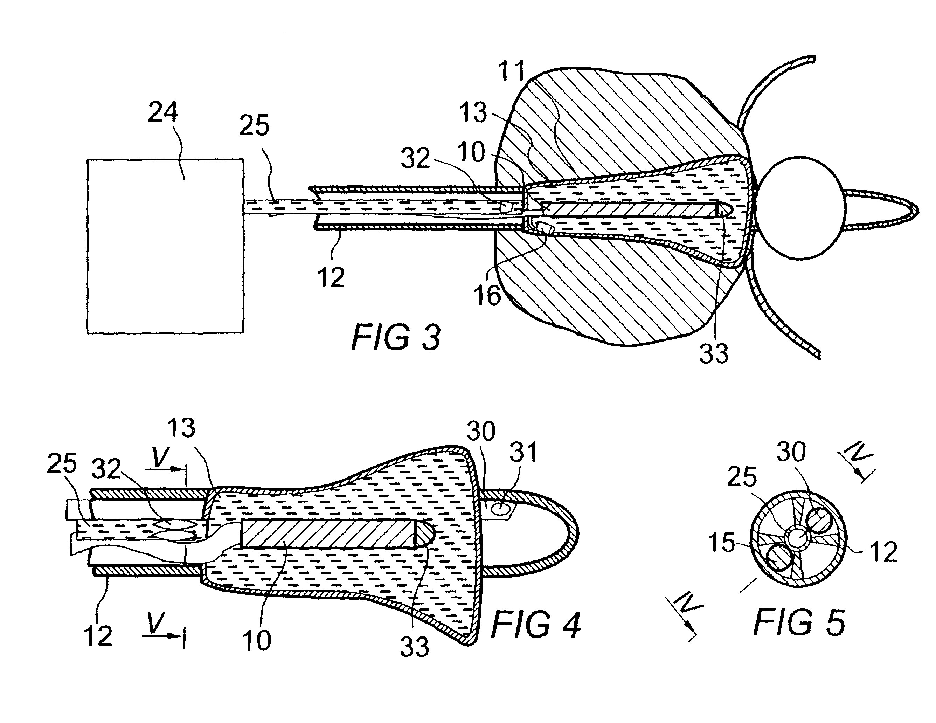

A heating device 10 is also contained in receptacle 11. It is in good thermal contact with fluid 13 which absorbs heat through convection from the heating device 10. Heat is then conveyed through the receptacle wall to the tissue that surrounds the receptacle. The heating device 10 is operatively connected through, among other things, a cable 15, to an energy supply unit 14 which is arranged outside of the body. The energy supply unit 14, in turn, is connected to a control and indicator unit 17. In order to facilitate localization of receptacle 11 the heating device 10 may constitute a lighting element, for instance in the form of a light bulb. This will in particular apply in such cases where positioning of the treatment catheter 12 is done with optical auxiliaries, such as an endoscope. The heating device 10 may also include a special lighting member, reference being made to the description of Fig. 3.

In a simple design, the heating device 10 consists of a resistance wire heated through supply of electrical energy via cable 15. In an alternate design, the heating device 10 comprises an element, which allows for both heating and cooling, such as some kind of a Peltier element. A great advantage with this latter design is that the treatment can be rapidly brought to an end by reversing the direction of the current to the Peltier element, resulting in a cooling effect. Fluid 13 therefore can be swiftly cooled to such a temperature that receptacle 11 can be removed from the body without the occurrence of any heating effect or injury to the tissue during passage through the body.

An important factor in heat treatment of this kind is the temperature generated in the tissue. In the configuration illustrated in Fig. 1, a first temperature transducer 16 is provided inside of receptacle 11. Temperature transducer 16 is operatively connected to control unit 17 to transmit temperature measurement results. These are used during the treatment procedure for controlling the energy supply unit 14 and are available to be viewed on the monitor display of indicator unit 17. In a practical design the temperature transducer 16 consists of a thermistor. There are also other types of temperature transducers, for example thermocouples and optical transmitters. There are also treatments for which the type of temperature indications generated by the illustrated temperature transducer 16 is not required. In such a case there is no need for this kind of transducer.

In the illustrated configuration, there is also provided a second temperature transducer 18. This transducer, however, is arranged outside of the area of treatment, displaced in the longitudinal direction of the treatment catheter 12. In a preferred embodiment the second temperature transducer 18 is provided in conjunction with catheter 12 and, specifically, inside of it. This temperature transducer as well may be designed in various ways. One advantage of using a temperature transducer outside of the treatment area is that it enables monitoring of the temperature of such tissue that is not to be treated. In many applications it is crucial that temperature increase in such tissue be minimal in order to avoid damage. Several designs of temperature transducers are presented in the description below with reference to Fig. 2. In an alternate embodiment not further detailed herein, the second temperature transducer 18 is provided externally on the treatment catheter 12 between expandable receptacles in the form of an inner balloon and an outer balloon. By inflating the inner balloon the temperature transducer 18 is displaced radially in the outward direction away from the center of treatment catheter 12, bringing the transducer by way of the outer balloon into engagement with the desired tissue.

The extent of receptacle 11 in the longitudinal direction of the catheter is 2 - 6 cm depending on the size of the concerned area of treatment. For treatment of other tissue than that of the prostate gland the dimensions may be completely different. Fluid 13 in the receptacle is heated by the heating device 10 to such a temperature that the surrounding tissue is heated to approximately 60 °C. At this temperature, treatment time will be about 1 hour. The treatment time can be selected to be both longer and shorter depending on the size of the treatment area and selected treatment temperature. By increasing the treatment temperature to the range of 90 - 150 °C, the treatment time can be decreased by a few minutes, such as by 5 minutes for instance. At these high temperatures the tissue hardens and forms a shell. This shell may prevent, or lessen, problems which might occur if the prostate gland undergoes swelling in connection with the treatment.

Upon completion of treatment the energy supply to the heating device 10 is discontinued and the receptacle is allowed to regain normal body temperature. As indicated above, decrease of the receptacle temperature can be achieved faster by the use of a combined cooling and heating element. The first temperature transducer 16 also has an important function during the conclusion of the treatment. It is unsuitable to remove the treatment catheter as long as the receptacle has such a temperature that damage can occur during the receptacle passage through the body. The temperature of the receptacle 11 therefore is continuously registered so that removal of the treatment catheter can take place as soon as the desired temperature has been reached.

In the case of treatment applied to the prostate or urine bladder and when catheter 12 is inserted in the urethra with the apex located in the urine bladder 21, drainage of urine and other liquid can be implemented through a drainage channel 30 provided in catheter 12. The drainage channel 30 runs through the entire catheter 12 and, with an opening 31, debouches near the apex of catheter 12. For certain kinds of treatment it may be suitable to leave the catheter 12 in place for some time upon treatment. Also during that time the drainage channel 30 upholds the function of draining the bladder of urine.

Upon completion of treatment in the prostate, the prostate gland tissue will swell, possibly leading to difficulties for the patient to urinate in such cases when the treatment catheter 12 is removed directly upon treatment accomplished. To prevent such difficulties from arising the treatment catheter can be equipped with a superposed, so called stent. Such a stent is schematically shown at 20 in Fig. 2. Stent 20 is a sleeve-like device, which is left behind in the prostatic urethra upon treatment and, thus, maintains the urine passage through the prostate gland. In a preferred embodiment the stent is provided to accompany the treatment catheter during insertion into the body, and then to remain in place both during the treatment and after its completion, at which point in time its intended usage begins. The dimensions of the treatment catheter relative to the inner dimensions of the stent are chosen so that the stent is not pulled out during removal of the catheter.

In a preferred embodiment the stent is composed of a bioabsorbable material and is absorbed some time after its insertion in the body. There is therefore no need for post-treatment in this respect. It is also possible to have the stent made from other material, for instance metal, and to remove the stent on a later occasion, if so required, when the tissue has regained its normal condition. The stent, for a certain kind of treatment, may also be inserted upon a completed treatment after withdrawal of the treatment catheter. For most types of prostate treatment the stent should be absorbed in the tissue or removed within 2 - 26 weeks upon treatment. The stent can also be designed for example as a soft tubing which, upon completion of treatment, is fixed along the prostatic urethra within the prostate gland in such a manner that it prevents the swollen tissue from cutting off the urine flow, thus maintaining a patent opening for urine outlet. The Figure shows an embodiment according to the invention with a stent 20 in the form of a helical winding of a thread of a suitable material. Stent 20 is arranged externally on the catheter 12 and is, in this configuration, shown inserted together with the catheter. Stent 20 comprises a thread of material which is absorbable in the tissue. The thread is wound in an helical or screw line configuration. Preferably, the inner diameter of stent 20 is somewhat greater than the outer diameter of catheter 12, and the stent 20 can therefore glide relatively free externally on the catheter. The outer diameter of stent 20 is greater than the inner dimensions of the urethra through the prostate gland so that stent 20 is secured to the proper extent in the prostate gland. Stent 20 may also be designed as a tube, net or fabric. In general the stent is shrunk at ambient temperature, but it can also be cooled before insertion. When it comes into contact with a warm tissue, it expands and is thereby fixated.

The stent 20 is passive during the treatment and does not affect the treatment outcome in any way. Upon accomplished treatment the balloon 22 is emptied. The catheter 12 is then withdrawn for removal leaving the stent behind in its place which prevents the prostate gland swelling from cutting off the urine flow during the time following treatment.

The axial extent of the tube or stent must not exceed the length of the prostate gland, which applies to both of the designs above, so as to not preclude the function of the external sphincter located in immediate connection to the rear portion of the prostate gland.

Upon treatment of the prostate three different complementary measures, in principle, may need to be resorted to in case of problems related to the urine passage. According to a first form of treatment, the treatment catheter is removed completely, and a new drainage catheter put in place if so needed. A second form of treatment involves usage of a stent. Most advantageously, the stent is entrained during insertion of the treatment catheter, but it is also possible to insert and position the stent afterwards. According to a third form of treatment, the treatment catheter is left behind in place after the actual treatment. In such a case the treatment catheter is designed with a drainage channel and functions as a conventional drainage catheter.

In certain cases it may be preferable that fixation of the stent 20 or tube is implemented with the aid of a special tool in direct conjunction with the final step of the treatment procedure. If non-absorbable tubing is used, such tubing has to be removed manually as soon as the swelling of the tissue has leveled off. This can be carried out with a special tool by gripping the tubing and then pulling it out.

Figure 2 shows a device according to the invention which involves improved heat transfer between the receptacle and the tissue to be treated. An element 19, which is expandable through temperature increase, is enclosed in the fluid receptacle 11 to augment the dimensions of the fluid receptacle 11 in the transverse direction of catheter 12 during temperature increase in the fluid receptacle 11. Thereby the abutment of receptacle 11 to the tissue in the area of treatment is enhanced as well as the heat transfer. Element 19 is preferably made of a material with a great coefficient of thermal expansion or has other properties involving expansion in connection with the treatment. Various types of memory metal, bimetal or similar can also be used. In the shown configuration, element 19 is in direct thermal contact with the heating device 10 so as to achieve a good coefficient of heat expansion in conjunction with heating of device 10.

Heating of the tissue takes place in the course of the ongoing treatment and should occur within certain temperature intervals so as to render the best possible treatment results. In order to allow for direct registration of temperature increase in the tissue that is to be treated, a third temperature transducer 26 is connected to a first carrier 27. Carrier 27 extends through a channel in catheter 12 and is configured such that it can be advanced through an opening in catheter 12. Preferably, there is provided a guide for carrier 27 in the catheter opening, directing carrier 27 out and into the tissue at the desired angle relative to catheter 12.

Either the carrier 27 or the temperature transducer 26 is equipped with a tip, which allows for a more simplified insertion into the tissue. Temperature transducer 26 may be either conventionally designed as a resistive transducer or a semi-conductor. The cable drawing required for such transducers is carried out through additional channels in catheter 12. If an optical type of transducer is used, a fiber optic conductor is provided through a channel in catheter 12.

Advancement of the temperature transducer 26 or its carrier 27 out of catheter 12 is controlled by control means from the exterior of the catheter outside of the body. This should preferably occur in a well defined way so that insertion into the tissue is implemented down to the desired depth. In a simple design, a rigid wire is provided through a channel in catheter 12. Temperature transducer 26 is provided at one end of the wire, and at the other end the wire is equipped with a handle. The channel and wire, which is contained in the channel, are given such dimensions and such resistance to bend that the degree of advancement becomes well defined in relation to the longitudinal advancement of the wire. The advancement by maneuvering of the handle and wire is limited by a stop or some arresting means so as to avoid the risk of the temperature sensing means 11 passing beyond the desired area of temperature sensing.

By continuous sensing of the temperature in the tissue being treated, it is possible to accurately control supplied power and, thus, the outcome of the treatment. The risk of undesired damage to the tissue is then significantly diminished.

In order to further lessen the risk of damage, and more specifically in such surrounding tissue which shall not be affected by treatment, a fourth temperature transducer 28 is connected to a second carrier 29. This second carrier 29 is designed to be advanced through and out of catheter 12 at a certain distance from the first carrier 27 in the longitudinal direction of the catheter 12. The distance is determined by the size of the treatment area and is ample enough to allow the fourth temperature transducer 28, in its forwardly advanced state, to penetrate into or abut against such a tissue which should not be damaged during treatment. In an alternate embodiment, not detailed herein, the fourth temperature transducer 28 is arranged externally on the treatment catheter 12 between an inner balloon and an outer balloon. By inflating the inner balloon, temperature transducer 28 is radially moved in the outward direction away from the center of the treatment catheter 12, thus enabling the transducer to come into engagement with the desired tissue.

In the device of Figure 3, receptacle 11 is connected via a channel 25 to a supply container 24 provided outside of the body. Receptacle 11 is filled from supply container 24, when the treatment catheter 12 has been placed correctly, and is locked through balloon 22. Filling is carried out until the desired pressure has been achieved in receptacle 11. This pressure also corresponds to an expansion of receptacle 11 which provides desired pressure against the surrounding tissue. The expansion causes receptacle 11 to take the shape of the trumpet or funnel shown in Fig. 3, the wider portion being closest to the neck of the urine bladder. Upon completion of treatment, receptacle 11 is cooled to body temperature, fluid 13 is vacated into the supply container 24, and balloon 22 is emptied. The catheter can then be pulled out. The configuration illustrated in Fig. 3 is very suitable in a combination with a stent 20. During vacation of fluid 13 from receptacle 11, the receptacle collapses. A stent, which has been provided externally on receptacle 11, will remain in the area of treatment after the treatment catheter 12 is withdrawn. Advantageously, there is no supply or drainage of fluid 13 during the actual heat treatment. The risk of tissue being unintentionally heated along catheter 12 is thereby reduced. Channel 25 is preferably provided centrally in catheter 12 to further lessen the risk of heat emission to such surrounding tissue that is not to be treated. For certain cases of treatment, however, it may be appropriate to supply fluid 13 during treatment to make receptacle 11 expand and press aside tissue already treated.

In the device as per Fig. 3, there is also provided a valve 32 in channel 25 near receptacle 11. Valve 32 assists in preventing the heated fluid from flowing backwards in channel 25 during treatment. In addition, valve 32 is used for maintaining desired pressure in receptacle 11.

The heating device 10 is supplemented with a special lighting element 33, which facilitates localization and placement of the treatment device. Light produced by lighting member 33 or by the heating device 10 itself may be visible light, or radiation of other suitable frequency.

In the device illustrated in Figure 4, valve 32 is designed as a one-way lip valve, which is open for fluid supply to receptacle 11, and through which a drainage catheter can be inserted from the outside when receptacle 11 is to be emptied. In a preferred configuration, however, valve 32 is provided at the catheter end outside of the body, and is then most suitably designed as a membrane, for example of rubber. The membrane is penetrated with a needle or a tube during filling or drainage of receptacle 11. Valve 32 may also be designed in various other ways.

For other types of treatment, for instance heat treatment, as a complement to other treatment of cancer in the urine bladder, receptacle 11 is composed of so called balloon silicone, i.e. a pliable material. Catheter 12 is advanced into the urethra to such an extent that the entire receptacle 11 is contained in the urine bladder. In this position, receptacle 11 is made to expand, causing engagement with a greater portion of the inside of the urine bladder, or with the entire urine bladder interior. Drainage can take place through the drainage channel 30 during the entire treatment.

## Claims

1. A device for heat treatment of prostate gland tissue, comprising a catheter (12) insertable into the prostate through urethra and having an expandable fluid receptacle (11) formed to be positioned in the prostate gland, wherein the fluid receptacle (11) contains heating means (10) for convective heating of fluid contained in receptacle (11) and, thus, heating the tissue in an area of treatment in the environment surrounding the fluid receptacle (11), and wherein the heating means (10) is connected to an energy supply unit (14) located outside of the body for supplying energy to the heating means (10) through the catheter (12), ***characterised* in.**
**that** a temperature transducer (26) is connected to a first carrier (27) extending through a channel in said catheter (12) to be movable from a first position inside of the treatment catheter (12) to a radially advanced second position outside of the treatment catheter (12), so as to allow said temperature transducer (26) to continuously sensing the temperature in the tissue being treated.

2. A device according to claim 1, **characterized in that** said heating means (10) comprises a heating element, which is heated electrically, and that said energy supply unit (14) is designed to supply the heating element with electric current.

3. A device according to claim 2, **characterized in that** said heating means comprises an electric resistance circuit.

4. A device as claimed in claim 1, **characterized in that** a first temperature transducer (16) is provided in the fluid receptacle (11) for continuously measuring the fluid temperature, and that said first temperature transducer (16) is operatively connected to a control unit (17), which in turn is operatively connected to said energy supply unit (14) in order to control it in dependence on the temperature of the fluid in receptacle (11) measured by the first temperature transducer (16).

5. A device as claimed in claim 1, **characterized in that** fluid (13) comprises water or physiological sodium chloride.

6. A device as claimed in claim 1, **characterized in that** the fluid receptacle (11) is a closed unit.

7. A device as claimed in claim 1, **characterized in that** a second temperature transducer (18) is provided which is displaced in the longitudinal direction of catheter (12) from the fluid receptacle (11) for measuring the temperature outside of the area of treatment.

8. A device according to any one of claims 1 - 7, **characterized in that** a stent (20) is releasably arranged over the fluid receptacle (11) when catheter (12) is inserted in the body.

9. A device as claimed in claim 1, **characterized in that** an element (19), which is expandable through temperature increase, is enclosed in the fluid receptacle (11) to augment the dimensions of the fluid receptacle (11) in the transverse direction of catheter (12) during temperature increase in the fluid receptacle (11) in order to enlarge the abutment of receptacle (11) to the tissue in the area of treatment.

10. A device according to claim 9, **characterized in that** the expandable element (19) comprises a body of bimetal thermally connected to the heating means (10).

11. A device as claimed in claim 9, **characterized in that** said expandable element (19) comprises a body of memory metal thermally connected to the heating means (10), said body adopting a first shape within a first temperature interval and a second shape within a second temperature interval.

12. A device as claimed in claim 1, **characterized in that** a drainage channel (30) for urine is provided, extending through the treatment catheter (12) and having an opening (31) near the tip of treatment catheter (12).

13. A device as claimed in claim 1, **characterized in that** a fluid channel (25), which is provided in the treatment catheter (12), is connected to receptacle (11) for both supply and drainage of fluid (13), and **in that** the receptacle (11) upon supply of fluid (13) is designed to adopt the shape of a larger cross-sectional area **in that** portion of the receptacle that is faced away from the fluid channel (25).

14. A device according to claim 13, **characterized in that** a valve (32) is provided in the fluid channel (25) located at the catheter end outside of the body.

15. A device as claimed in claim 13, **characterized in that** a valve (32) is provided in the fluid channel (25) near the receptacle (11).

16. A device according to any one of the preceding claims, **characterized in that** lighting means (33) is provided internal to receptacle (11).

17. A device according to any one of the preceding claims, **characterized in that** said heating means (10) comprises lighting means (33).

18. A device as claimed in claim 2, **characterized in that** said heating element comprises a Peltier element (21).

## Patentansprüche

1. Vorrichtung zur Wärmebehandlung von Prostatagewebe umfassend einen Katheter (12), der in die Prostata durch die Harnröhre einsetzbar ist und ein expandierbares Flüssigkeitsbehältnis (11) aufweist, welches so geformt ist, daß es in der Prostata positioniert werden kann, wobei das Flüssigkeitsbehältnis (11) ein Heizmittel (10) zur konvektiven Erwärmung der in dem Behältnis (11) enthaltenen Flüssigkeit enthält und dadurch das Gewebe in einem Behandlungsbereich in der den Flüssigkeitsbehälter (11) umschließenden Umgebung erwärmt, und wobei das Heizmittel (10) mit einer außerhalb des Körpers angeordneten Energieversorgungseinheit (14) zur Energieversorgung der Heizmittel (10) durch den Katheter (12) verbunden ist, **dadurch gekennzeichnet,**
**daß** ein Temperaturwandler (26) mit einem ersten sich durch einen Kanal in dem Katheter (12) erstreckenden Träger (27) verbunden ist,
um bewegbar von einer ersten Position im Inneren des Behandlungskatheters (12) zu einer radial hervorgehobenen zweiten Position außerhalb des Behandlungskatheters (12) zu sein, um es so dem Temperaturwandler (26) zu ermöglichen, kontinuierlich die Temperatur in dem zu behandelnden Gewebe abzufühlen.

2. Vorrichtung nach Anspruch 1, **dadurch gekennzeichnet, daß** das Heizmittel (10) ein Heizelement umfaßt, welches elektrisch geheizt wird, und daß die Energieversorgungseinheit (14) ausgelegt ist, um das Heizelement mit elektrischem Strom zu versorgen.

3. Vorrichtung nach Anspruch 2, **dadurch gekennzeichnet, daß** das Heizmittel eine elektrische Widerstandsschaltung umfaßt.

4. Vorrichtung nach Anspruch 1, **dadurch gekennzeichnet, daß** ein erster Temperaturwandler (16) in dem Flüssigkeitsbehältnis (11) zur kontinuierlichen Messung der Flüssigkeitstemperatur vorgesehen ist, und daß der erste Temperaturwandler (16) mit einer Steuerungseinheit (17) wirksam verbunden ist, die wiederum mit der Energieversorgungseinheit (14) wirksam verbunden ist, um sie in Abhängigkeit von der Temperatur der Flüssigkeit in dem Behältnis (11), die von dem ersten Temperaturwandler (16) gemessen wird, zu steuern.

5. Vorrichtung nach Anspruch 1, **dadurch gekennzeichnet, daß** die Flüssigkeit (13) Wasser oder physiologisches Natriumchlorid umfaßt.

6. Vorrichtung nach Anspruch 1, **dadurch gekennzeichnet, daß** der Flüssigkeitsbehälter (11) eine geschlossene Einheit ist.

7. Vorrichtung nach Anspruch 1, **dadurch gekennzeichnet, daß** ein zweiter Temperaturwandler (18) vorgesehen ist, der in der Längsrichtung des Katheters (12) von dem Flüssigkeitsbehälter (11) zur Messung der Temperatur außerhalb des Behandlungsbereichs versetzt angeordnet ist.

8. Vorrichtung nach einem der Ansprüche 1 bis 7, **dadurch gekennzeichnet, daß** ein Stent (20) lösbar über dem Flüssigkeitsbehältnis (11) angeordnet ist, wenn der Katheter (12) in den Körper eingesetzt wird.

9. Vorrichtung nach Anspruch 1, **dadurch gekennzeichnet, daß** ein Element (19), welches durch Temperaturerhöhung expandierbar ist, in das Flüssigkeitsbehältnis (11) eingeschlossen ist, um die Abmessungen des Flüssigkeitsbehältnisses (11) in der transversalen Richtung des Katheters (12) während der Temperaturerhöhung in dem Flüssigkeitsbehältnis (11) zu vergrößern, damit das Anstoßen des Behältnisses (11) an das Gewebe im Behandlungsbereich verstärkt wird.

10. Vorrichtung nach Anspruch 9, **dadurch gekennzeichnet, daß** das expandierbare Element (19) einen Körper aus Bimetall umfaßt, der thermisch mit den Heizmitteln (10) verbunden ist.

11. Vorrichtung nach Anspruch 9, **dadurch gekennzeichnet, daß** das expandierbare Element (19) einen Körper aus Memory-Metall umfaßt, der thermisch mit den Heizmitteln (10) verbunden ist, wobei dieser Körper eine erste Gestalt innerhalb eines ersten Temperaturintervalls und eine zweite Gestalt innerhalb eines zweiten Temperaturintervalls annimmt.

12. Vorrichtung nach Anspruch 1, **dadurch gekennzeichnet, daß** ein Drainagekanal (30) für Urin vorgesehen ist, der sich durch den Behandlungskatheter (12) erstreckt und eine Öffnung (31) in der Nähe der Spitze des Behandlungskatheters (12) aufweist.

13. Vorrichtung nach Anspruch 1, **dadurch gekennzeichnet, daß** ein Flüssigkeitskanal (25), der in dem Behandlungskatheter (12) vorgesehen ist, mit dem Behältnis (11) sowohl für die Versorgung als auch für die Drainage von Flüssigkeit (13) verbunden ist, und daß das Behältnis (11) ausgelegt ist, nach Zuführung von Flüssigkeit (13) die Form eines größeren Querschnittbereichs in demjenigen Teil des Behältnisses anzunehmen, der von dem Flüssigkeitskanal (25) abgewandt ist.

14. Vorrichtung nach Anspruch 13, **dadurch gekennzeichnet, daß** am Ende des Katheters außerhalb des Körpers ein Ventil (32) in dem Flüssigkeitskanal (25) vorgesehen ist.

15. Vorrichtung nach Anspruch 13, **dadurch gekennzeichnet, daß** ein Ventil (32) in dem Flüssigkeitskanal (25) in der Nähe des Behältnisses (11) vorgesehen ist.

16. Vorrichtung nach einem der vorstehenden Ansprüche, **dadurch gekennzeichnet, daß** ein Beleuchtungsmittel (33) im Inneren des Behältnisses (11) vorgesehen ist.

17. Vorrichtung nach irgendeinen der vorstehenden Ansprüche, **dadurch gekennzeichnet, daß** das Heizmittel (10) das Beleuchtungsmittel (33) umfaßt.

18. Vorrichtung nach Anspruch 2, **dadurch gekennzeichnet, daß** das Heizelement ein Peltier-Element (21) umfaßt.

## Revendications

1. Dispositif pour le traitement par la chaleur de tissu de la prostate, comprenant un cathéter (12) susceptible d'être inséré dans la prostate par l'urètre et ayant un réceptacle de fluide extensible (11) formé de manière à être positionné dans la prostate, lequel réceptacle de fluide (11) contient un moyen de chauffage (10) pour chauffage par convection du fluide contenu dans le réceptacle (11) et donc pour chauffer le tissu dans la zone de traitement dans l'environnement entourant le réceptacle de fluide (11) et lequel moyen de chauffage (10) est relié à une unité d'alimentation en énergie (14) située à l'extérieur du corps pour fournir de l'énergie au moyen de chauffage (10) par le cathéter (12), **caractérisé en ce que**
un transducteur de température (26) est relié à un premier porteur (27) s'étendant dans un canal dans ledit cathéter (12) pour pouvoir être déplacé depuis une première position à l'intérieur du cathéter de traitement (12) vers une seconde position avancée radialement à l'extérieur du cathéter de traitement (12), de manière à permettre au dit transducteur de température (26) de capter continuellement la température dans le tissu en cours de traitement.

2. Dispositif selon la revendication 1, **caractérisé en ce que** ledit moyen de chauffage (10) comprend un élément de chauffage, qui est chauffé électriquement, et **en ce que** ladite unité d'alimentation en énergie (14) est conçue pour alimenter l'élément de chauffage en courant électrique.

3. Dispositif selon la revendication 2, **caractérisé en ce que** ledit moyen de chauffage comprend un circuit de résistance électrique.

4. Dispositif selon la revendication 1, **caractérisé en ce qu'**un premier transducteur de température (16) est fourni dans le réceptacle de fluide (11) pour mesurer continuellement la température du fluide et **en ce que** ledit premier transducteur de température (16) est relié de manière fonctionnelle à une unité de contrôle (17) qui à son tour est reliée de manière fonctionnelle à ladite unité d'alimentation en énergie (14) afin de la contrôler en fonction de la température du fluide dans le réceptacle (11) mesurée par le premier transducteur de température (16).

5. Dispositif selon la revendication 1, **caractérisé en ce que** le fluide (13) comprend de l'eau ou de la solution physiologique de chlorure de sodium.

6. Dispositif selon la revendication 1, **caractérisé en ce que** le réceptacle de fluide (11) est une unité fermée.

7. Dispositif selon la revendication 1, **caractérisé en ce qu'**un second transducteur de température (18) est fourni qui est déplacé en direction longitudinale du cathéter (12) depuis le réceptacle de fluide (11) pour mesurer la température en dehors de la zone de traitement.

8. Dispositif selon l'une quelconque des revendications 1 à 7, **caractérisé en ce qu'**un stent (20) est arrangé de manière à pouvoir être libéré sur le réceptacle de fluide (11) lorsque le cathéter (12) est inséré dans le corps.

9. Dispositif selon la revendication 1, **caractérisé en ce qu'**un élément (19), qui est extensible par une augmentation de la température, est inclus dans le réceptacle de fluide (11) pour augmenter les dimensions du réceptacle de fluide (11) dans la direction transversale du cathéter (12) pendant l'augmentation de la température dans le réceptacle de fluide (11) afin d'agrandir la butée du réceptacle (11) jusqu'au tissu dans la zone de traitement.

10. Dispositif selon la revendication 9, **caractérisé en ce que** l'élément extensible (19) comprend un corps de bimétal connecté thermiquement au moyen de chauffage (10).

11. Dispositif selon la revendication 9, **caractérisé en ce que** ledit élément extensible (19) comprend un corps de métal à mémoire relié thermiquement au moyen de chauffage (10), ledit corps adoptant une première forme dans un premier intervalle de température et une seconde forme dans un second intervalle de température.

12. Dispositif selon la revendication 1, **caractérisé en ce qu'**un canal de drainage (30) pour l'urine est fourni, qui s'étend à travers le cathéter de traitement (12) et qui a une ouverture (31) proche de l'embout du cathéter de traitement (12).

13. Dispositif selon la revendication 1, **caractérisé en ce qu'**un canal de fluide (25), qui est fourni dans le cathéter de traitement (12), est relié au réceptacle (11) pour l'apport et le drainage de fluide (13) et **en ce que** le réceptacle (11) lors de l'apport de fluide (13) est conçu pour adopter la forme d'une zone de section transversale plus importante dans la portion du réceptacle qui est dos au canal de fluide (25).

14. Dispositif selon la revendication 13, **caractérisé en ce qu'**une valve (32) est fournie dans le canal de fluide (25) situé à l'extrémité du cathéter en dehors du corps.

15. Dispositif selon la revendication 13, **caractérisé en ce qu'**une valve (32) est fournie dans le canal de fluide (25) près du réceptacle (11).

16. Dispositif selon l'une quelconque des revendications précédentes, **caractérisé en ce qu'**un moyen d'éclairage (33) est fourni à l'intérieur du réceptacle (11).

17. Dispositif selon l'une quelconque des revendications précédentes, **caractérisé en ce que** ledit moyen de chauffage (10) comprend un moyen d'éclairage (33).

18. Dispositif selon la revendication 2, **caractérisé en ce que** ledit élément de chauffage comprend un élément de Peltier (21).
